# EUROPEAN PATENT SPECIFICATION

(11) **EP 2 494 998 B1**
(45) Date of publication and mention of the grant of the patent: **03.12.2014**
(21) Application number: 10826482.1
(22) Date of filing: 05.10.2010
(51) Int. Cl.: A61M 1/14, G01N 27/06, A61M 1/16, G01N 27/10

(54) **METHOD FOR MONITORING DIALYSATE CONCENTRATION AND DEVICE THEREFOR**
VERFAHREN ZUR ÜBERWACHUNG EINER DIALYSATKONZENTRATION UND VORRICHTUNG DAFÜR
PROCÉDÉ DE SURVEILLANCE DE LA CONCENTRATION EN DIALYSAT ET DISPOSITIF POUR SA MISE EN OEUVRE

(30) Priority: 29.10.2009 JP 2009249001
(43) Date of publication of application: 05.09.2012
(73) Proprietor: Nipro Corporation, Osaka-shi, Osaka 531-8510 (JP); Shibuya Kogyo Co., Ltd., Kanazawa-shi, Ishikawa 920-8681 (JP)
(72) Inventor: YANAGIMOTO Yoji, Osaka-shi Osaka 531-8510 (JP); SAWADA Toshiharu, Kanazawa-shi Ishikawa 920-8681 (JP); MATSUMOTO Yasuhisa, Kanazawa-shi Ishikawa 920-8681 (JP); MISHIMA Takashi, Kanazawa-shi Ishikawa 920-8681 (JP)
(74) Representative: Oxley, Rachel Louise
(86) International application number: PCT/JP2010/067402
(87) International publication number: WO 2011/052348

(56) References cited:
- EP-A2- 0 160 272
- WO-A1-92/05814
- WO-A1-93/09822
- JP-A- 6 197 956
- JP-A- 10 201 842
- JP-A- 11 262 521
- JP-A- H06 504 708
- JP-A- 2007 117 396
- US-A- 4 895 657
- US-A- 5 900 136

## Description

### Technical Field

The present invention relates to a dialysate concentration monitoring method and apparatus, and more particularly, to a dialysate concentration monitoring method and apparatus which measure electrical conductivity of dialysate and monitor the concentration of the dialysate by comparing the electrical conductivity with a predetermined reference value.

### Background Art

Conventionally, personal dialysis apparatus are designed to prepare dialysate by mixing liquid A, liquid B, and dilution water, and supply the prepared dialysate to a dialyzer. Also, the personal dialysis apparatus are designed to constantly monitor the concentration of the dialysate, and stop the supply and output a warning when the concentration falls out of a predetermined range.

Known methods for monitoring dialysate concentration include a method which measures the concentration of the dialysate based on electrical conductivity. The method involves installing electrical conductivity measuring means in the dialysis apparatus, setting a reference value for the electrical conductivity of the dialysate prepared in advance, comparing measured values obtained by the measuring means with the reference value, and thereby monitoring whether or not the dialysate has a predetermined concentration (Patent Literature 1).

According to Patent Literature 1, by inputting concentrations of components contained in liquid A and liquid B, the reference value for the electrical conductivity of the dialysate to be prepared is calculate based on the component concentrations and a mixing ratio.

Incidentally, liquid A described above is prepared by dissolving agent A composed mainly of an electrolyte and glucose in water while liquid B is prepared by dissolving agent B composed principally of sodium hydrogen carbonate in water. RO water, i.e., water purified by passage through a reverse osmosis membrane is used as the dilution water.

EP0160272 describes an apparatus for monitoring dialysate concentration. It has (a) means for measuring electrical conductivity of the dialysate to monitor the concentration of the dialysate, and (b) means to alter the concentration of a dialysate component.

### Prior Art Documents

### Patent Literature

Patent Literature 1: Japanese Patent No. 3319756

### Summary of Invention

### Problems to be Solved by the Invention

The reference value for the electrical conductivity of dialysate needs to be changed simultaneously when the concentration of the dialysate to be prepared is changed, and conventionally an operator of the dialysis apparatus resets the reference value, for example, by referring to a table of correspondence between dialysate concentration and electrical conductivity.

However, the electrical conductivity of dialysate varies with the mixing ratio between liquid A and liquid B even when the concentration of the dialysate remains the same, and it takes a great deal of effort to measure the electrical conductivity of each of various conceivable concentration patterns in advance. Beside, the reference value cannot be reset during dialysis treatment.

Also, according to Patent Literature 1 described above, although a new reference value can be calculated by changing the mixing ratio, the concentrations of components serving as a basis for calculation of the reference value are nominal values rather than actually measured values, and thus the calculated reference value can sometimes differ from actually measured values of the electrical conductivity of prepared dialysate.

In view of the above problems, the present invention provides a dialysate concentration monitoring method and apparatus which make it easy to set the reference value for electrical conductivity according to concentration of dialysate to be prepared and allow concentration changes to be made even during dialysis treatment.

### Means for Solving the Problems

That is, according to claim 1 of the present invention, there is provided a dialysate concentration monitoring method which measures electrical conductivity of dialysate prepared by mixing liquid A, liquid B, and dilution water and monitors concentration by comparing the electrical conductivity with a predetermined reference value, the method comprising: determining a ratio of changes in the electrical conductivity of the dialysate to concentration changes of liquid A alone and a ratio of changes in the electrical conductivity of the dialysate to concentration changes of liquid B alone; determining the electrical conductivity of the dialysate to be prepared, in accordance with concentration of liquid A and concentration of liquid B in the dialysate to be prepared, based on the ratios of the electrical conductivity of the dialysate to the concentration changes of liquid A and liquid B; and setting the reference value based on the electrical conductivity.

Also, according to claim 3 of the present invention, there is provided a dialysate concentration monitoring apparatus installed on a dialysis apparatus which prepares dialysate by mixing liquid A, liquid B, and dilution water according to a set mixing ratio, provided with conductivity measuring means for measuring electrical conductivity of the dialysate, and adapted to monitor concentration by comparing the measured electrical conductivity with a predetermined reference value, the dialysate concentration monitoring apparatus comprising: concentration adjusting means for adjusting mixing amounts of liquid A, liquid B, and dilution water in the dialysate; and setting changing means for changing a setting of the reference value, wherein the conductivity measuring means measures the electrical conductivity of the dialysate in a plurality of cases in which the concentration adjusting means changes concentration of liquid A alone as well as changes concentration of liquid B alone in the dialysate, the setting changing means determines a ratio of changes in the electrical conductivity of the dialysate to concentration changes of liquid A alone as well as a ratio of changes in the electrical conductivity of the dialysate to concentration changes of liquid B alone, and when dialysate with a different mixing ratio among liquid A, liquid B, and dilution water is newly prepared in the dialysis apparatus, the setting changing means determines the electrical conductivity of the dialysate to be newly prepared, in accordance with concentration of liquid A and concentration of liquid B in the dialysate to be newly prepared, based on the ratios of the electrical conductivity of the dialysate to the determined concentration changes of liquid A and liquid B and sets the reference value based on the determined electrical conductivity.

### Advantageous Effects of Invention

As described above, according to claim 1 and claim 3 of the present invention, the electrical conductivity of the dialysate to be prepared can be found by determining a ratio of changes in the electrical conductivity of the dialysate to concentration changes of liquid A alone as well as a ratio of changes in the electrical conductivity of the dialysate to concentration changes of liquid B alone.

Consequently, in dialysate concentration monitoring, the reference value for electrical conductivity can be set easily according to the concentration of dialysate to be prepared and concentration changes can be made even during dialysis treatment.

### Brief Description of Drawings

[Figure 1] Figure 1 is a perspective view of a dialysis apparatus according to an embodiment of the present invention.
[Figure 2] Figure 2 is a liquid circuit diagram of the dialysis apparatus according to the present embodiment.
[Figure 3] Figure 3 is a table showing actually measured values of electrical conductivity obtained by varying concentrations of liquid A and liquid B.
[Figure 4] Figure 4 is a graph showing ratios of changes in electrical conductivity to concentration changes of liquid A and liquid B.

### Mode for Carrying out the Invention

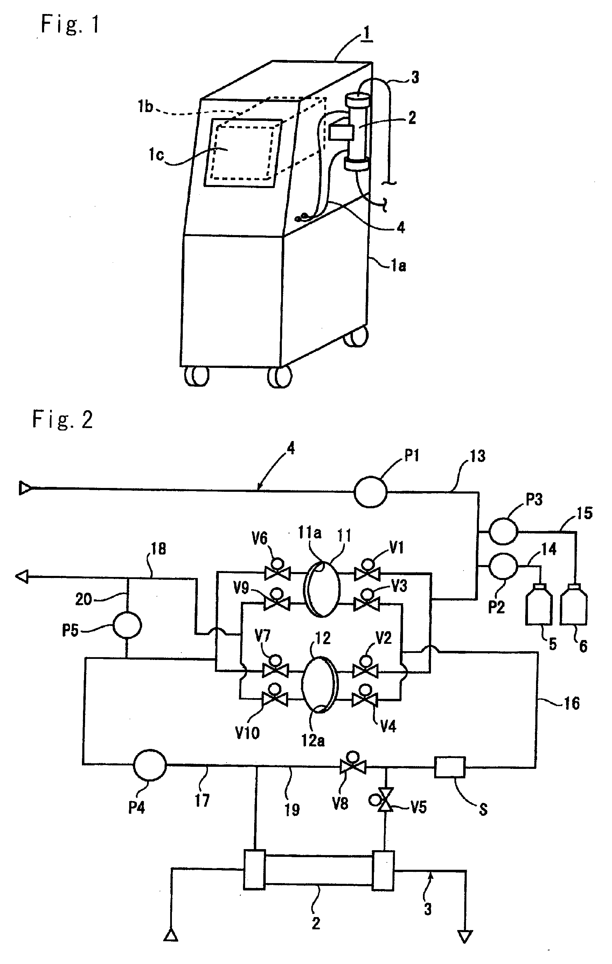

To describe an illustrated embodiment, Figure 1 shows a general view of a dialysis apparatus 1 and Figure 2 shows a liquid circuit installed inside the dialysis apparatus 1, wherein the dialysis apparatus 1 is a personal dialysis apparatus designed to prepare dialysate and administer dialysis treatment to a single patient.

The dialysis apparatus 1 includes a dialyzer 2 held to a main body 1a, a blood circuit 3 connected to the dialyzer 2, a dialysate circuit 4 installed inside the main body 1a, and control means 1b which makes up a concentration monitoring apparatus.

The control means 1b is provided with a touch panel input unit 1c which accepts necessary operations to allow an operator set the concentration of the dialysate used in dialysis treatment by operating the input unit 1c.

Hollow threads (not shown) are installed in the dialyzer 2. The inside of the hollow threads is communicated with the blood circuit 3 to allow blood to flow therethrough while space outside the hollow threads is communicated with the dialysate circuit 4 to allow the dialysate to flow therethrough in a direction opposite the blood.

The dialysate circuit 4 shown in Figure 2 is connected with water supply means (not shown) adapted to supply clear water such as RO water as dilution water, a liquid-A container 5 adapted to supply liquid A which is an undiluted solution of the dialysate, a liquid-B container 6 adapted to supply liquid B which is an undiluted solution, and a drainage tube (not shown) adapted to discharge used dialysate.

The dialysis apparatus 1 is designed to prepare dialysate by mixing the clear water supplied by the water supply means, liquid A supplied from the liquid-A container 5, and liquid B supplied from the liquid-B container 6, supply dialysate to the dialyzer 2, and thereby control dialysis treatment, using the dialysate circuit 4.

The dialysate circuit 4 includes two chambers, i.e., a first and second chambers 11 and 12, a water supply channel 13 connected to the first and second chambers 11 and 12 and adapted to allow clear water to flow from the water supply means, a liquid-A supply channel 14 connected to the water supply channel 13 and adapted to allow liquid A to flow from the liquid-A container 5, a liquid-B supply channel 15 connected to the water supply channel 13 and adapted to allow liquid B to flow from the liquid-B container 6, an unused-dialysate channel 16 installed between the first and second chambers 11 and 12 and the dialyzer 2 and adapted to allow unused dialysate to flow through, a used-dialysate channel 17 installed between the first and second chambers 11 and 12 and the dialyzer 2 and adapted to allow used dialysate to flow through, and a drainage channel 18 adapted to discharge the used dialysate from the first and second chambers 11 and 12 to the drainage tube.

The first and second chambers 11 and 12 have identical shapes and have their interiors partitioned into two rooms each by respective diaphragms 11a and 12a. The spaces on one side (right side in Figure 2) are connected with the water supply channel 13 and unused-dialysate channel 16 while the spaces on the other side (left side in Figure 2) are connected with the used-dialysate channel 17 and drainage channel 18.

A clear water pump P1 is installed on the water supply channel 13 to adjust the feed rate of clear water under the control of the control means 1b. The clear water pump P1 is connected to the first and second chambers 11 and 12 by branching off in two directions in a downstream portion of the water supply channel 13, with solenoid valves V1 and V2 being installed on respective branch channels.

The liquid-A supply channel 14 is connected to the water supply channel 13 on the upstream side of the branch channels and equipped with a liquid-A pump P2 which is capable of adjusting the feed rate of liquid A under the control of the control means 1b.

The liquid-B supply channel 15 is connected to the water supply channel 13 on the upstream side of the connecting position of the liquid-A supply channel 14 and equipped with a liquid-B pump P3 which is capable of adjusting the feed rate of liquid B under the control of the control means 1b.

The clear water pump P1, liquid-A pump P2, and liquid-B pump P3 make up concentration adjusting means provided in the concentration monitoring apparatus and adapted to adjust mixing amounts of liquid A, liquid B, and dilution water in the dialysate.

The unused-dialysate channel 16 is connected to the first and second chambers 11 and 12 by branching off in two directions in its upstream portion, with solenoid valves V3 and V4 being installed on respective branch channels.

Also, a conductivity sensor S and solenoid valve V5 are installed on the unused-dialysate channel 16, where the conductivity sensor S serves as conductivity measuring means adapted to measure the electrical conductivity of the dialysate prepared in the first and second chambers 11 and 12 while the solenoid valve V5 is installed downstream of the conductivity sensor S and adapted to close the unused-dialysate channel.

The used-dialysate channel 17 is connected to the first and second chambers 11 and 12 by branching off in two directions in its downstream portion, with solenoid valves V6 and V7 being installed on respective branch channels.

Also, a dialysate pump P4 adapted to deliver the dialysate in the dialyzer 2 is installed on the used-dialysate channel 17. The dialysate pump P4 is designed to adjust a flow rate of the dialysate flowing through the dialyzer 2, under the control of the control means 1b.

Furthermore, a defective-dialysate discharge channel 19 is disposed between that part of the unused-dialysate channel 16 on which the conductivity sensor S and solenoid valve V5 are installed and that part of the used-dialysate channel 17 which is located upstream of the dialysate pump P4. A solenoid valve V8 is installed on the defective-dialysate discharge channel 19.

The drainage channel 18 is connected to the first and second chambers 11 and 12 by branching off in two directions in its upstream portion, with solenoid valves V9 and V10 being installed on respective branch channels.

A water removal channel 20 is disposed between the used-dialysis channel 17 and drainage channel 18 and a water removal pump P5 is installed on the water removal channel 20. When the water removal pump P5 is operated during dialysis treatment, the dialysate correspond to the removed amount of water is drained directly to the drainage channel 18.

In the dialysis apparatus 1 configured as described above, when the solenoid valve V1 on the side of the first chamber 11 on the water supply channel 13 is opened and the solenoid valve V2 on the side of the second chamber 12 is closed and the solenoid valve V3 on the side of the first chamber 11 on the unused-dialysate channel 16 is closed and the solenoid valve V4 on the side of the second chamber 12 is opened, the solenoid valve V6 on the side of the first chamber 11 on the used-dialysate channel 17 is closed, the solenoid valve V7 on the side of the second chamber 12 is opened, the solenoid valve V9 on the side of the first chamber 11 on the drainage channel 18 is opened, and the solenoid valve V10 on the side of the second chamber 12 is closed.

In this state, the clear water pump P1 delivers clear water from the water supply means at a predetermined feed rate. At the same time, the liquid-A pump P2 delivers liquid A from the liquid-A container 5 at a predetermined feed rate and the liquid-B pump P3 delivers liquid B from the liquid-B container 6 at a predetermined feed rate.

Consequently, clear water, liquid A, and liquid B flow into one of the spaces in the first chamber 11 at a predetermined ratio and are mixed to form unused dialysate of a preset concentration.

Along with the inflow of the clear water, liquid A, and liquid B into the one of the spaces in the first chamber 11, the used dialysate which has been pooled in the other space in the first chamber 11 is pushed out into the drainage channel 18 via the diaphragm 11a and discharged to the drainage tube.

On the other hand, the dialysate pump P4 delivers the used dialysate from the used-dialysate channel 17 at a predetermined flow rate, causing the unused dialysate prepared in one of the spaces in the second chamber 12 to be supplied to the dialyzer 2 via the unused-dialysate channel 16. At the same time, the used dialysate flows into the other space in the second chamber 12 from the dialyzer 2.

Open/closed states of the solenoid valves V1, V2, V3, V4, V6, V7, V9, and V10 are designed to be switched at each lapse of a predetermined time, causing dialysate to be prepared alternately in the first chamber 11 and second chamber 12 and supplied therefrom.

The conductivity sensor S serving as conductance measuring means installed on the unused-dialysate channel 16 measures the electrical conductivity of the unused dialysate flowing into the unused-dialysate channel 16. Then, the control means 1b serving as a concentration monitoring apparatus compares the measured electrical conductivity with a preset reference value and thereby determines whether or not the concentration of the unused dialysate is appropriate.

When the electrical conductivity of the unused dialysate is, for example, within 5% tolerances for the reference value, the control means 1b determines that the concentration of the unused dialysate is appropriate.

On the other hand, if an error in excess of ±5% is detected, the control means 1b determines that the concentration of the unused dialysate is abnormal, outputs warning instructions, and brings up a warning display on the input unit 1c. Also, the control means 1b closes the solenoid valve V5 and opens the solenoid valve V8 to discharge the unused dialysate to the defective-dialysate discharge channel 19 and used-dialysate channel 17.

In this way, the dialysis apparatus 1 is designed to administer dialysis treatment by supplying clear water, liquid A, and liquid B to the first chamber 11 and second chamber 12, preparing dialysate, and supplying the dialysate to the dialyzer 2 while at the same time measuring and thereby monitoring the concentration and electrical conductivity of the prepared dialysate.

The conductivity sensor S adapted to measure the electrical conductivity of the dialysate and the control means 1b adapted to compare the measured electric conductance with the reference value make up the dialysate concentration monitoring apparatus.

The dialysis apparatus 1 according to the present embodiment is designed such that the concentration of the dialysate to be prepared can be set on a patient by patient basis and that the operator can enter a plurality of concentration patterns differing in concentration of liquid A and concentration of liquid B and timing for changing the concentration in the input unit 1c of the dialysis apparatus 1 in order to change the concentration of the dialysate during dialysis treatment.

Again, the concentration monitoring apparatus is designed to automatically change the reference value for concentration monitoring and monitor the changed concentration of the dialysate.

Thus, the concentration monitoring apparatus according to the present embodiment is provided with setting changing means adapted to change the reference value. Using the setting changing means, the concentration monitoring apparatus determines the electrical conductivity of the dialysate to be newly prepared, in accordance with concentration of liquid A and concentration of liquid B and then sets a new reference value. The control means 1b is designed to monitor the concentration of the dialysate prepared based on the new reference value.

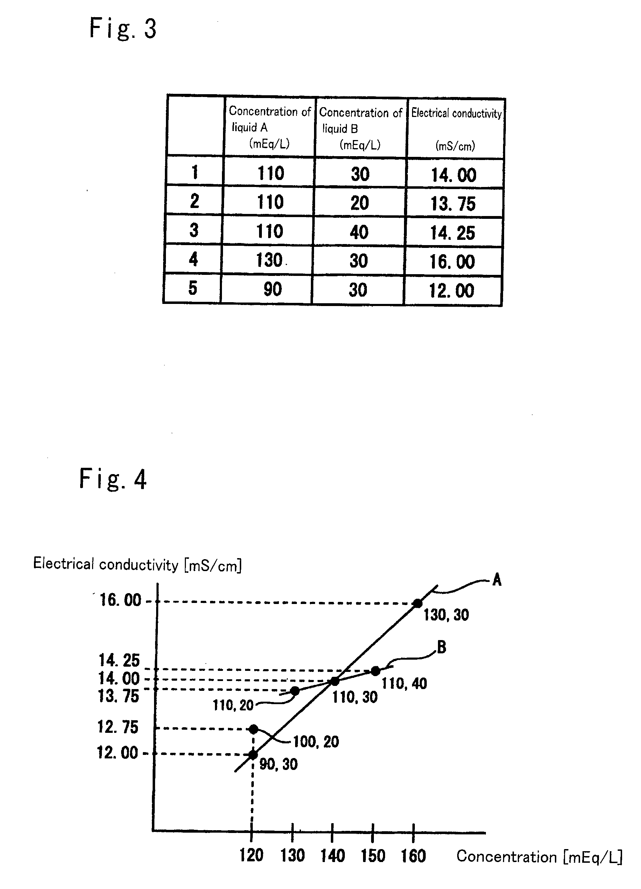

A method for setting the reference value for use in concentration monitoring carried out by the concentration monitoring apparatus will be described below. Figure 3 shows a settings screen displayed on the input unit 1c of the dialysis apparatus 1, and settings are supposed to be made before a dialysis treatment.

Starting from the left, the table displayed on the screen shows sample Nos. 1 to 5, concentration of liquid A (mEq/L), concentration of liquid B (mEq/L), and electrical conductivity (mS/cm) of the dialysate containing the indicated concentrations of liquid A and liquid B. Also, fields are provided at the bottom of the table to enable entering concentrations of liquid A and liquid B of the dialysate used during dialysis treatment.

Values of sample No. 1 are basic concentrations and basic electrical conductivity. The basic concentrations of liquid A and liquid B of sample No. 1 are those entered by the operator via the input unit 1c while the basic electrical conductivity is an actually measured value of the dialysate prepared based on the concentration of liquid A and the concentration of liquid B.

According to the present embodiment, the values of the dialysate entered at the start of treatment are used as the basic concentrations of liquid A and liquid B, but values registered in advance with the control means 1b may be used alternatively.

Based on the basic concentrations of liquid A and liquid B, the concentration adjusting means automatically sets concentrations of liquid A and liquid B of sample Nos. 2 to 4.

Specifically, the values of sample Nos. 2 and 3 are obtained by varying only the concentration of liquid B by predetermined amounts from the basic concentration of sample No. 1: the concentration of liquid B of sample No. 2 is 10 mEq/L smaller than the basic concentration and the concentration of liquid B of sample No. 3 is 10 mEq/L larger than the basic concentration.

Also, the values of sample Nos. 4 and 5 are obtained by varying only the concentration of liquid A by predetermined amounts from the basic concentration of sample No. 1: the concentration of liquid A of sample No. 4 is 20 mEq/L larger than the basic concentration and the concentration of liquid A of sample No. 5 is 20 mEq/L smaller than the basic concentration.

Preferably, the values set by the concentration adjusting means fall within a range which includes the concentration of liquid A and the concentration of liquid B of the dialysate prepared during dialysis treatment.

After setting the concentrations of liquid A and liquid B of sample Nos. 2 to 5, the concentration adjusting means prepares dialysate in the first and second chambers 11 and 12 before the treatment by controlling the clear water pump P1, liquid-A pump P2, and liquid-B pump P3, based on the concentrations of liquid A and liquid B of sample No. 1.

After being discharged from the first and second chambers 11 and 12, the dialysate flows through the unused-dialysate channel 16 and has its electrical conductivity measured by the conductivity sensor S. Then, the control means 1b registers the electrical conductivity of the dialysate with the setting changing means as the basic electrical conductivity of sample No. 1 and displays the electrical conductivity on the screen.

Regarding sample Nos. 2 to 5, dialysates are prepared based on the respective concentrations of liquid A and liquid B. The electrical conductivity of each of the prepared dialysates is measured, and the measured values are registered with the setting changing means and displayed on the screen.

Next, regarding sample Nos. 1, 4, and 5 in the table of Figure 3, which are different in the value of the concentration of liquid A, but equal in the value of the concentration of liquid B, the setting changing means finds the ratio of changes in the electrical conductivity of the dialysate to concentration changes of liquid A based on the values of the concentration of liquid A and the measured values of electrical conductivity.

Similarly, regarding sample Nos. 1, 2, and 3 in Figure 3, which are different in the value of the concentration of liquid B, but equal in the value of the concentration of liquid A, the setting changing means finds the ratio of changes in the electrical conductivity of the dialysate to concentration changes of liquid B based on the values of the concentration of liquid B and the measured values of electrical conductivity.

Figure 4 is a graph in which the abscissa represents concentration of dialysate calculated by adding the concentration of liquid A and the concentration of liquid B while the ordinate represents the electrical conductivity.

In Figure 4, the slope of straight line A obtained from the values of sample Nos. 1, 4, and 5 which differ only in the concentration of liquid A corresponds to the ratio of changes in the electrical conductivity of the dialysate to concentration changes of liquid A.

Similarly, the slope of straight line B obtained from the values of sample Nos. 1, 2, and 3 which differ only in the concentration of liquid B corresponds to the ratio of changes in the electrical conductivity of the dialysate to concentration changes of liquid B.

According to the present invention, the reference value for use in concentration monitoring can be set by determining the electrical conductivity of the dialysate prepared based on the ratios of changes in the electrical conductivity thus obtained.

Specifically, the electrical conductivity of the dialysate to be prepared is found by setting the measured basic electrical conductivity and correcting the basic electrical conductivity based on the ratios of changes. In the case of Figure 4, the slope of straight line A is 0.1 and the slope of straight line B is 0.025.

Next, description will be given of an example of how the electrical conductivity of the dialysate to be prepared is found by correcting the basic electrical conductivity based on the ratios of changes in the electrical conductivity of the dialysate to concentration changes of liquid A and liquid B.

Using the example of Figures 3 and 4, it is assumed here that when the basic concentration of liquid A is 110 mEq/L, basic concentration of liquid B is 30 mEq/L, and basic electrical conductivity is 14.00 mS/cm in dialysate, dialysate in which the concentration of liquid A is 110 mEq/L and concentration of liquid B is 20 mEq/L is newly prepared.

First, the setting changing means determines a difference between the basic concentration and new concentration of liquid A. In this case, the concentration difference is -10 mEq/L.

Next, based on the determined concentration difference of liquid A and the ratio (the slope of straight line A) of changes in the electrical conductivity of the dialysate to concentration changes of liquid A alone, the value of electrical conductivity to be increased or decreased from the basic electrical conductivity is calculated.

That is, in the above example, when the concentration of liquid A changes -10 mEq/L, the electrical conductivity, which changes by an amount equal to the concentration change of liquid A multiplied by 0.1, changes -1 mS/cm from the basic electrical conductivity.

Next, the setting changing means determines a difference between the basic concentration and new concentration of liquid B. In this case, the concentration difference is -10 mEq/L.

Next, based on the determined concentration difference of liquid B and the ratio (the slope of straight line B) of changes in the electrical conductivity of the dialysate to concentration changes of liquid B alone, the value of electrical conductivity to be increased or decreased from the basic electrical conductivity is calculated.

That is, in the above example, when the concentration of liquid B changes -10 mEq/L, the electrical conductivity, which changes by an amount equal to the concentration change of liquid B multiplied by 0.025, changes -0.25 mS/cm from the basic electrical conductivity.

Finally, the basic electrical conductivity is corrected based on amounts of changes in the electrical conductivity corresponding to the concentration changes of liquid A and liquid B determined above. In the above example, 12.75 mS/cm is obtained as a new value of electrical conductivity by subtracting 1.00 mS/cm for liquid A and 0.25 mS/cm for liquid B from the basic electrical conductivity of 14.00 mS/cm.

Then, the setting changing means sets the reference value for use in concentration monitoring of the dialysate to be newly prepared, based on the determined electrical conductivity and registers the reference value with the control means 1b serving as the concentration monitoring apparatus. Then, the control means 1b monitors the dialysate based on the reference value.

In this way, according to the present embodiment, when predetermined basic concentrations are set on the concentration monitoring apparatus, first the electrical conductivity of the dialysate is calculated based on the basic concentrations and the calculated electrical conductivity is set as basic electrical conductivity.

Next, the difference between the basic concentration of liquid A and the concentration of liquid A in the dialysate to be newly prepared is determined, and then the value of electrical conductivity to be increased or decreased from the basic electrical conductivity is determined based on the ratio of changes in the electrical conductivity of the dialysate to concentration changes of liquid A alone.

Furthermore, the difference between the basic concentration of liquid B and the concentration of liquid B in the dialysate to be newly prepared is determined, and then the value of electrical conductivity to be increased or decreased from the basic electrical conductivity is determined based on the ratio of changes in the electrical conductivity of the dialysate to concentration changes of liquid B alone.

Then, the basic electrical conductivity is corrected based on the amounts of changes (values of increases or decreases) in the electrical conductivity corresponding to the concentration changes of liquid A and liquid B determined above, and the electrical conductivity of the dialysate to be prepared is determined.

In this way, as the operator enters concentration of liquid A and concentration of liquid B of desired dialysate in the input unit 1c, the control means 1b determines the electrical conductivity of the desired dialysate and automatically sets the reference value for use in concentration monitoring.

This makes it possible to change the concentration of dialysate even during dialysis treatment.

Also, since the ratios of changes in the electrical conductivity of the dialysate to concentration changes of liquid A and liquid B are determined based on values sampled by actually measuring the electric conductivities of multiple dialysates using liquid A and liquid B currently in use, accurate electrical conductivity of the dialysate can be determined as quickly as possible.

### Reference Signs List

- 1: Dialysis apparatus
- 4: Dialysate circuit
- 6: Liquid-B container
- 14: Liquid-A supply channel
- 16: Unused-dialysate channel
- 1b: Control means
- 5: Liquid-A container
- 13: Water supply channel
- 15: Liquid-B supply channel
- S: Conductivity sensor

## Claims

1. A dialysate concentration monitoring method which measures electrical conductivity of dialysate prepared by mixing liquid A, liquid B, and dilution water and monitors concentration by comparing the electrical conductivity with a predetermined reference value, the method comprising:
determining a ratio of changes in the electrical conductivity of the dialysate to concentration changes of liquid A alone and a ratio of changes in the electrical conductivity of the dialysate to concentration changes of liquid B alone;
determining the electrical conductivity of the dialysate to be prepared, in accordance with concentration of liquid A and concentration of liquid B in the dialysate to be prepared, based on the ratios of the electrical conductivity of the dialysate to the concentration changes of liquid A and liquid B; and
setting the reference value based on the electrical conductivity.

2. The dialysate concentration monitoring method according to claim 1, further comprising:
setting basic electrical conductivity of dialysate containing liquid A and liquid B of predetermined concentrations;
correcting the basic electrical conductivity based on the ratios of the electrical conductivity of the dialysate to the concentration changes of liquid A and liquid B; and
thereby determining the electrical conductivity of the dialysate to be prepared.

3. A dialysate concentration monitoring apparatus installed on a dialysis apparatus (1) which prepares dialysate by mixing liquid A, liquid B, and dilution water according to a set mixing ratio, provided with conductivity measuring means (S) for measuring electrical conductivity of the dialysate, and adapted to monitor concentration by comparing the measured electrical conductivity with a predetermined reference value, the dialysate concentration monitoring apparatus comprising:
concentration adjusting means (P1, P2, P3) for adjusting mixing amounts of liquid A, liquid B, and dilution water in the dialysate; and
setting changing means (1b) for changing a setting of the reference value,
wherein the conductivity measuring means (S) measures the electrical conductivity of the dialysate in a plurality of cases in which the concentration adjusting means (P1, P2, P3) changes concentration of liquid A alone as well as changes concentration of liquid B alone in the dialysate,
the setting changing means (1b) determines a ratio of changes in the electrical conductivity of the dialysate to concentration changes of liquid A alone as well as a ratio of changes in the electrical conductivity of the dialysate to concentration changes of liquid B alone, and
when dialysate with a different mixing ratio among liquid A, liquid B, and dilution water is newly prepared in the dialysis apparatus (1), the setting changing means (1b) determines the electrical conductivity of the dialysate to be newly prepared, in accordance with concentration of liquid A and concentration of liquid B in the dialysate to be newly prepared, based on the ratios of the electrical conductivity of the dialysate to the determined concentration changes of liquid A and liquid B and sets the reference value based on the determined electrical conductivity.

## Patentansprüche

1. Dialysatkonzentrationsüberwachungsverfahren, das elektrische Leitfähigkeit von Dialysaten misst, die durch Mischen von Flüssigkeit A, Flüssigkeit B und Verdünnungswasser hergestellt werden, und die Konzentration überwacht, indem die elektrische Leitfähigkeit mit einem vorbestimmten Vergleichswert verglichen wird, wobei das Verfahren Folgendes umfasst:
Bestimmen eines Verhältnisses von Veränderungen der elektrischen Leitfähigkeit des Dyalisats zu Veränderungen der Konzentration von Flüssigkeit A alleine und eines Verhältnisses von Veränderungen der elektrischen Leitfähigkeit des Dialysats zu Veränderungen der Konzentration von Flüssigkeit B alleine;
Bestimmen der elektrischen Leitfähigkeit des herzustellenden Dialysats entsprechend der Konzentration von Flüssigkeit A und der Konzentration von Flüssigkeit B im herzustellenden Dialysat, basierend auf den Verhältnissen der elektrischen Leitfähigkeit des Dialysats zu den Veränderungen der Konzentration von Flüssigkeit A und Flüssigkeit B; und
Einstellen des Vergleichswerts basierend auf der elektrischen Leitfähigkeit.

2. Dialysatkonzentrationsüberwachungsverfahren nach Anspruch 1, ferner umfassend:
Einstellen einer elektrischen Basisleitfähigkeit des Dialysats, das Flüssigkeit A und Flüssigkeit B in vorbestimmten Konzentrationen enthält;
Korrigieren der elektrischen Basisleitfähigkeit basierend auf den Verhältnissen der elektrischen Leitfähigkeit des Dialysats zu den Veränderungen der Konzentration von Flüssigkeit A und Flüssigkeit B; und
dadurch Bestimmen der elektrischen Leitfähigkeit des herzustellenden Dialysats.

3. Dialysatkonzentrationsüberwachungsvorrichtung, installiert auf einer Dialysevorrichtung (I), die ein Dialysat durch Mischen von Flüssigkeit A, Flüssigkeit B und Verdünnungswasser gemäß einem bestimmten Mischverhältnis herstellt, bereitgestellt mit einem Leitfähigkeitsmessmittel (S) zum Messen der elektrischen Leitfähigkeit des Dialysats, und angepasst für die Überwachung der Konzentration durch Vergleichen der gemessenen elektrischen Leitfähigkeit mit einem vorbestimmten Vergleichswert, wobei die Dialysatkonzentrationsüberwachungsvorrichtung Folgendes umfasst:
ein Konzentrationseinstellungsmittel (P1, P2, P3) zum Einstellen von Mischmengen von Flüssigkeit A, Flüssigkeit B und Verdünnungswasser im Dialysat; und
ein Feststellungsänderungsmittel (1 b) zum Ändern einer Festlegung des Vergleichswerts,
wobei das Leitfähigkeitsmessmittel (S) die elektrische Leitfähigkeit des Dialysats in einer Vielzahl von Fällen misst, in denen das Konzentrationseinstellungsmittel (P1, P2, P3) die Konzentration von Flüssigkeit A allein sowie die Konzentration von Flüssigkeit B allein im Dialysat verändert,
wobei das Feststellungsänderungsmittel (1 b) ein Verhältnis von Veränderungen der elektrischen Leitfähigkeit des Dialysats zu Veränderungen der Konzentration von Flüssigkeit A allein sowie ein Verhältnis von Veränderungen der elektrischen Leitfähigkeit des Dialysats zu Veränderungen der Konzentration von Flüssigkeit B alleine bestimmt, und wobei,
wenn ein Dialysat mit einem unterschiedlichen Mischverhältnis von Flüssigkeit A, Flüssigkeit B und Verdünnungswasser in der Dialysevorrichtung (1) neu hergestellt wird, das Feststellungsänderungsmittel (1 b) die elektrische Leitfähigkeit des neu herzustellenden Dialysats entsprechend der Konzentration von Flüssigkeit A und der Konzentration von Flüssigkeit B im neu herzustellenden Dialysat basierend auf den Verhältnissen der elektrischen Leitfähigkeit des Dialysats zu den bestimmten Veränderungen der Konzentration von Flüssigkeit A und Flüssigkeit B bestimmt und den Vergleichswert basierend auf der bestimmten elektrischen Leitfähigkeit festlegt.

## Revendications

1. Procédé de surveillance de concentration de dialysat qui mesure la conductivité électrique d'un dialysat préparé en mélangeant un liquide A, un liquide B et une eau de dilution et qui surveille la concentration en comparant la conductivité électrique avec une valeur de référence prédéterminée, le procédé comprenant :
la détermination d'un rapport entre les changements de conductivité électrique du dialysat et les variations de concentration du liquide A seul et d'un rapport entre les changements de la conductivité électrique du dialysat et les variations de concentration du liquide B seul ;
la détermination de la conductivité électrique du dialysat à préparer, en fonction de la concentration du liquide A et de la concentration du liquide B dans le dialysat à préparer, sur la base des rapports entre la conductivité électrique du dialysat et les variations de concentration du liquide A et du liquide B ; et
le réglage de la valeur de référence sur la base de la conductivité électrique.

2. Procédé de surveillance de concentration de dialysat selon la revendication 1, comprenant en outre :
le réglage de la conductivité électrique de base du dialysat contenant le liquide A et le liquide B en des concentrations prédéterminées ;
la correction de la conductivité électrique de base sur la base des rapports entre la conductivité électrique du dialysat et les variations de concentration du liquide A et du liquide B ; et
la détermination, de ce fait, de la conductivité électrique du dialysat à préparer.

3. Appareil de surveillance de concentration de dialysat installé sur un appareil de dialyse (1) qui prépare un dialysat en mélangeant un liquide A, un liquide B et une eau de dilution selon un rapport de mélange fixé, pourvu de moyens de mesure de conductivité (S) pour mesurer la conductivité électrique du dialysat, et conçu pour surveiller la concentration en comparant la conductivité électrique mesurée avec une valeur de référence prédéterminée, l'appareil de surveillance de concentration de dialysat comprenant :
des moyens d'ajustement de concentration (P1, P2, P3) pour ajuster les quantités de mélange du liquide A, du liquide B et de l'eau de dilution dans le dialysat ; et
des moyens de modification de réglage (1b) pour modifier le réglage de la valeur de référence,
dans lequel les moyens de mesure de conductivité (S) mesurent la conductivité électrique du dialysat dans une pluralité de cas dans lesquels les moyens d'ajustement de concentration (P1, P2, P3) modifient la concentration du liquide A seul et modifient également la concentration du liquide B seul dans le dialysat,
les moyens de modification de réglage (1b) déterminent un rapport entre les changements de la conductivité électrique du dialysat et les variations de concentration du liquide A seul, ainsi qu'un rapport entre les changements de la conductivité électrique du dialysat et les variations de concentration du liquide B seul, et
lorsqu'un dialysat avec un rapport de mélange différent entre le liquide A, le liquide B et l'eau de dilution est nouvellement préparé dans l'appareil de dialyse (1), les moyens de modification de réglage (1b) déterminent la conductivité électrique du dialysat à préparer nouvellement, en fonction de la concentration de liquide A et de la concentration de liquide B dans le dialysat à préparer nouvellement, sur la base des rapports entre la conductivité électrique du dialysat et les variations de concentration déterminées du liquide A et du liquide B et règlent la valeur de référence sur la base de la conductivité électrique déterminée.
